# EUROPÄISCHE PATENTANMELDUNG

(11) **EP 1 262 471 A2**
(43) Veröffentlichungstag der Anmeldung: **04.12.2002**
(21) Anmeldenummer: 02010290.1
(22) Anmeldetag: 21.05.2002
(51) Int. Cl.: C07C 39/367, C07C 37/055, C07C 43/225, C07C 41/01, C07C 17/20, C07C 22/04

(54) **Fluor enthaltende Bisphenole, deren Herstellung, deren Vor- und Zwischenprodukte sowie deren Verwendung**

(30) Priorität: 31.05.2001 DE 10126432
(71) Anmelder: BAYER AG, 51368 Leverkusen (DE)
(72) Erfinder: Marhold, Albrecht, Dr., 51373 Leverkusen (DE); Baumann, Käthe, Dr., 42109 Wuppertal (DE)

(57) **Zusammenfassung**

Zur Verfügung gestellt werden neue Fluor enthaltende Bisphenole der allgemeinen Formel (I) 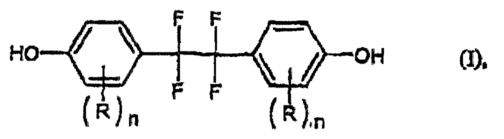 worin R und n die in der Beschreibung angegebenen Bedeutungen haben, ein Verfahren zu deren Herstellung sowie einige Vor- und Zwischenprodukte dieser Herstellung. Die Fluor enthaltenden Bisphenole sind besonders als Einsatzstoffe für die Herstellung von Flüssigkristallen, Polymeren und Flammschutzmitteln geeignet.

## Beschreibung

Die vorliegende Erfindung betrifft Fluor enthaltende Bisphenole, deren Herstellung, einige Vor- und Zwischenprodukte dieser Herstellung sowie die Verwendung der Fluor enthaltenden Bisphenole als Einsatzstoffe für die Herstellung von Flüssigkristallen, Polymeren und Flammschutzmitteln.

Bestimmte Fluor enthaltende Bisphenole sind grundsätzlich bereits als Ausgangsstoffe zur Herstellung von Flüssigkristallen (Polym. Mater. Sci. Eng. **1996**, 74, 133-134) oder als Monomere zur Polymerisierung (Fluoropolymers **1999**, 1, 127-150), beispielsweise zur Herstellung von Polycarbonat (JP 05170892 A2), Polyethern (JP 2000273166 A2) oder Polyestern (Polymer **1997**, 38, 3669-3676) bekannt. Es ist ferner bekannt, dass es bei Flüssigkristallen von Vorteil ist, wenn diese eine lineare Molekülstruktur besitzen (C. Weygand, "Chemische Morphologie der Flüssigkeiten", Handbuch und Jahrbuch der chemischen Physik, Band 2, Abschnitt 3c, Leipzig, Akadem. Verlagsges. 1941; H.-G. Elias, Makromoleküle, 5. Auflage, Band 1, Kapitel 20, Abschnitt 20.1.2, Hüthig & Wepf Verlag Basel 1990).

Aus J. Gen. Chem. USSR (Engl. Transl.), **1965**, 35, 1616-1623 ist es bekannt, dass die Herstellung von verschiedenen 1,2-Di(4-halophenyl)tetrachlorethanen (B) durch Kreuzkupplung von 4-Halobenzotrichloriden (A) mit Kupfer in Pyridin erfolgen kann. Dieses Verfahren liefert neben den gewünschten 1,2-Di(4-halophenyl)tetrachlorethanen der Formel (B) in gewissen Mengen auch 1,2-Di(4-halophenyl)dichlorethylene der Formel (C). Nachteilig ist, dass sich diese Verbindungen der Formel (C) nicht direkt zu den entsprechenden 1,2-Di(4-halophenyl)tetrafluorethanen fluorieren lassen, was zu kostspieligen Ausbeuteverlusten führt.

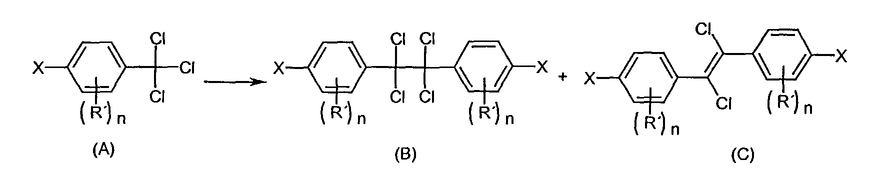

Eine Fluorierung von 1,2-Di(4-halophenyl)tetrachlorethan der Formel (B) zu 1,2-Di(4-halophenyl)tetrafluorethan (C) ist gemäß J. Gen. Chem. USSR (Engl. Transl.), **1965**, 35, 1616-1623 nur mit dem hochgiftigen und teuren Antimon(III)fluorid und einem Katalysator bei hohen Temperaturen möglich.

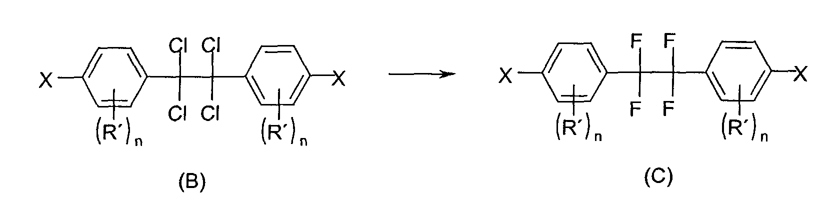

Aus US-A-4,168,388 ist ein Verfahren zur Herstellung von 2-, 3- und 4-Trifluormethylphenol aus dem entsprechenden 2-, 3- und 4-Trifluormethylchlorbenzol bekannt. Es hat den Nachteil, dass das leichtentzündliche und heftig mit Wasser reagierende Natriumhydrid als Base eingesetzt wird. Zusätzlich wurde gefunden, dass das eingesetzte Lösungsmittel N,N-Dimethylacetamid sich unter den beschriebenen Reaktionsbedingungen zu dem korrosiven N,N-Dimethylamin zersetzt. Ein Recycling des Lösungsmittels wird dadurch verhindert und das Verfahren kostspielig.

Aufgrund des zunehmenden Bedarfs an Flüssigkristallen bestand die Aufgabe der vorliegenden Erfindung darin, neue Fluor enthaltende bifunktionelle Verbindungen mit linearer Molekularstruktur bereitzustellen.

Gelöst wird diese Aufgabe durch 1,2-Di(4-hydroxyaryl)tetrafluorethane der allgemeinen Formel (I) 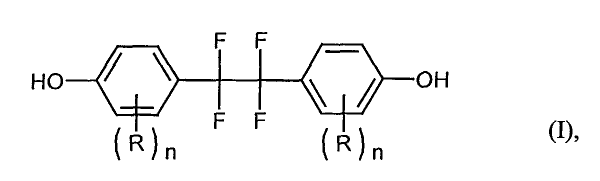 worin
- R: unabhängig voneinander Wasserstoff, F, Cl, Br, I, CN, COOR², C₁-C₄-Alkyl, C₁-C₄-Alkoxy, C₁-C₄-Alkylthio, C₁-C₄-Perfluoralkyl, C₁-C₄-Perfluoralkoxy, C₁-C₄-Perfluoralkylthio, C₁-C₄-Polyfluoralkyl, C₁-C₄-Polyfluoralkoxy und C₁-C₄-Polyfluoralkylthio bedeuten können,
- R²: C₁-C₄-Alkyl ist und
- n: eine ganze Zahl von 0 bis 4 ist.

Bevorzugt sind Verbindungen der allgemeinen Formel (I), worin
- R: unabhängig voneinander Wasserstoff, F, Cl, Br, Methyl, Methoxy, Trifluormethyl oder Trifluormethoxy bedeuten können und
- n: 0 oder 1 ist.

Besonders bevorzugt sind als Verbindungen der allgemeinen Formel (I) 1,2-Di(4-hydroxyphenyl)tetrafluorethan, 1,2-Di(3-chlor-4-hydroxyphenyl)tetrafluorethan, 1,2-Di(3-fluor-4-hydroxyphenyl)tetrafluorethan, 1,2-Di(3-brom-4-hydroxyphenyl)tetrafluorethan und 1,2-Di(3-methyl-4-hydroxyphenyl)-tetrafluorethan.

Gegenstand der Erfindung ist ferner ein Verfahren zur Herstellung der Verbindungen der allgemeinen Formel (I), welches dadurch gekennzeichnet ist, dass man Verbindungen der allgemeinen Formel (VI) 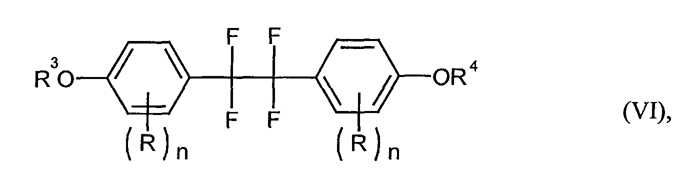 worin
- R³ und R⁴: gleich oder verschieden sind und Benzyl, substituiertes Benzyl, bevorzugt 1-(C₁-C₄-Alkyl)benzyl, Benzhydryl, substituiertes Benzhydryl, iso-Propyl, tert-Butyl oder Cyclohexyl darstellen und
- R und n: die gleiche Bedeutung besitzen wie in der allgemeinen Formel (I),
einer Etherspaltung unterwirft.

Die Etherspaltung dieser 1,2-Di(4-alkoxyphenyl)tetrafluorethane der Formel (VI) erfolgt entweder über eine Hydrierung oder eine Spaltung im sauren Milieu. Wenn R³ und/oder R⁴ einen Benzylrest oder substituierten Benzylrest darstellen, hat sich besonders die Hydrierung bewährt, für alle übrigen Bedeutungen von R³ und R⁴ ist die Spaltung im sauren Milieu die bevorzugte Variante. Sowohl die Hydrierung als auch die Spaltung im sauren Milieu können nach bekannten Methoden des Standes der Technik durchgeführt werden. Zur Spaltung im sauren Milieu werden üblicherweise wässriger Säuren wie beispielsweise HCl, HBr, H₂SO₄, Essigsäure oder Phosphorsäure eingesetzt. Die Hydrierung erfolgt unter Einsatz von Wasserstoff und herkömmlichen Hydrierkatalysatoren wie z.B. geträgerten oder ungeträgerten Edelmetallkatalysatoren. Geeignet ist beispielsweise Palladium auf Aktivkohle in einem organischen Lösungsmittel wie Ethanol.

Die Verbindungen der allgemeinen Formel (VI) sind bisher nicht bekannt. Gegenstand der Erfindung sind daher auch die Verbindungen der allgemeinen Formel (VI) 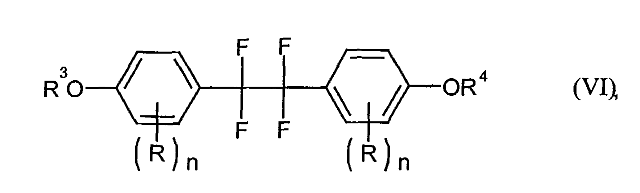 worin
- R³ und R⁴: gleich oder verschieden sind und Benzyl, substituiertes Benzyl, bevorzugt 1-(C₁-C₄-Alkyl)benzyl, Benzhydryl, substituiertes Benzhydryl, iso-Propyl, tert-Butyl oder Cyclohexyl darstellen und
- R und n: die gleiche Bedeutung besitzen wie in der allgemeinen Formel (I).

Sie können durch eine Veretherung mit den entsprechenden Alkoholen gewonnen werden.

Gegenstand der Erfindung ist daher ferner ein Verfahren zur Herstellung von Verbindungen der allgemeinen Formel (VI), dadurch gekennzeichnet, dass man Verbindungen der allgemeinen Formel (V) 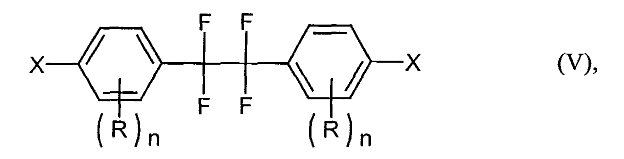 worin X für ein Halogen oder Pseudohalogen und R und n die für die allgemeine Formel (I) genannten Bedeutungen besitzen, mit einem Alkohol der Formel R³OH und/oder einem Alkohol der Formel R⁴OH umsetzt, wobei R³ und R⁴ die für die allgemeine Formel (VI) genannten Bedeutungen besitzen.

Bevorzugt werden bei diesem Verfahren Verbindungen der allgemeinen Formel (V) eingesetzt, bei denen X für Fluor oder Chlor steht. Als Alkohol wird bevorzugt Benzylalkohol eingesetzt.

Diese Reaktion des 1,2-Di(4-halophenyl)tetrafluorethans der allgemeinen Formel (V) zum 1,2-Di(4-alkoxyphenyl)tetrafluorethan der allgemeinen Formel (VI) erfolgt üblicherweise in Gegenwart einer anorganischen Base in einem polaren aprotischen Lösungsmittel.

Die anorganischen Base kann beispielsweise ein Hydroxid, Carbonat, Hydrogensulfat, Sulfat, Hydrogenphosphat oder Phosphat eines Alkali- oder Erdalkalimetalls sein. Bevorzugt wird Kaliumhydroxid eingesetzt.

Das polare aprotische Lösungsmittel kann erfindungsgemäß ein Amid, wie N,N-Dimethylacetamid oder N-Methylpyrrolidon, ein Sulfoxid, wie Dimethylsulfoxid, ein Sulfon, wie Tetramethylensulfon, oder ein Nitril, wie Acetonitril, sein. Vorzugsweise wird N-Methylpyrrolidon verwendet. Gegebenenfalls kann in Gegenwart von Wasser gearbeitet werden.

Am Ende der Reaktion kann das Lösungsmittel zum Teil oder vollständig durch Destillation (gegebenenfalls als Gemisch mit in der Reaktion entstehendem Wasser) wiedergewonnen werden. Wässriges N-Methylpyrrolidon kann beispielsweise noch mehrfach in weiteren Partien dieser Verfahrensstufe wiederverwendet werden, wobei keine Trocknung notwendig ist. Damit kann bei Einsatz von Kaliumhydroxid als Base der Abfall in dieser Reaktion auf Kaliumchlorid beschränkt werden.

Nach beendeter Reaktion und Abdestillieren des Lösungsmittels kann das so erhaltene Produkt beispielsweise durch Umkristallisieren oder Rühren in einem geeigneten Lösungsmittel, Abfiltrieren und Trocknen gereinigt werden.

Die Verbindungen der allgemeinen Formel (V) können durch eine Fluorierung der entsprechenden chlorierten Verbindungen hergestellt werden.

Gegenstand der Erfindung ist somit ferner ein Verfahren zur Herstellung von Verbindungen der allgemeinen Formel (V), dadurch gekennzeichnet, dass man Verbindungen der allgemeinen Formel (III) 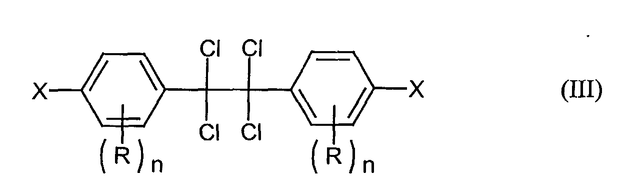 mit wasserfreier Fluorwasserstoffsäure umsetzt.

Hierbei werden üblicherweise 4 bis 50 Mol wasserfreie Fluorwasserstoffsäure pro Mol Verbindung (III) eingesetzt, wobei das im Handel unter der Bezeichnung "wasserfreie Fluorwasserstoffsäure" erhältliche Material ausreichend wasserfrei ist.

Die Fluorierung kann beispielsweise bei Temperaturen von 0 - 180°C und einem Druck im Bereich von 1 - 50 bar durchgeführt werden. Bevorzugt arbeitet man bei Temperaturen von 10 - 160°C und einem Druck von 10 - 30 bar. Gegebenenfalls erfolgt die Umsetzung in Gegenwart eines Katalysators und/oder eines inerten Lösungsmittels. Als Katalysator kann beispielsweise Bortrifluorid, Titantetrachlorid und Antimonpentachlorid und -fluorid verwendet werden, als Lösungsmittel hat sich Dichlormethan bewährt.

Man kann die wasserfreie Fluorwasserstoffsäure vorlegen und die Verbindung (III) zudosieren oder umgekehrt verfahren. Es ist vorteilhaft, die Fluorwasserstoffsäure und die Verbindung (III) im Rahmen der zuvor genannten Temperaturbereiche bei relativ niedrigen Temperaturen, z.B. bis zu 50°C, zu vereinen und danach die Temperatur in Stufen zu erhöhen. Gegebenenfalls kann die überschüssige wasserfreie Fluorwasserstoffsäure durch Abdestillieren nahezu vollständig wiedergewonnen werden.

Nach beendeter Reaktion und Abdestillieren der überschüssigen wasserfreien Fluorwasserstoffsäure ist es möglich, das Reaktionsgemisch umzukristallisieren oder mit einem geeigneten Lösungsmittel zu versetzen, z.B. mit Dichlormethan. Bei dieser Form der Aufarbeitung wird die organische Phase anschließend mit Aktivkohle und/oder einem Alkalimetallfluorid versetzt, filtriert und eingeengt oder wässrig aufgearbeitet. Das so erhaltene Produkt 1,2-Di(4-halophenyl)tetrafluorethan der allgemeinen Formel (V) kann beispielsweise durch Umkristallisieren oder Rühren in einem geeigneten Lösungsmittel, Abfiltrieren und Trocknen gereinigt werden.

Dieses Verfahren zeichnet sich im Vergleich zu dem aus J. Gen. Chem. USSR (Engl. Transl.), **1965**, 35, 1616-1623 bekannten Verfahren dadurch aus, dass anstelle großer Mengen des Antimontrifluorids Fluorwasserstoffsäure verwendet wird und Antimontrifluorid höchstens optional in sehr geringen Mengen als Katalysator zugesetzt wird. Hierdurch ist es möglich, das Verfahren im Hinblick auf die sicherheitstechnischen Vorkehrungen deutlich einfacher und in wirtschaftlich attraktiver Weise durchzuführen.

Die Verbindungen der allgemeinen Formel (III) können durch die Umsetzung von Benzotrichloriden der allgemeinen Formel (II) 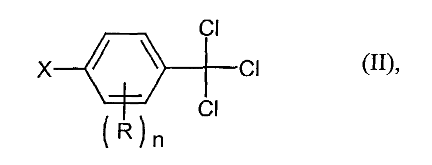 worin
- R und n: die für die allgemeine Formel (I) angegebenen Bedeutungen besitzen und
- X: ein Halogen oder Pseudohalogen ist,
in Gegenwart von Kupfer sowie in einem tertiären Amin als Lösungsmittel hergestellt werden.

Bei dieser Umsetzung handelt es sich um eine Kreuzkupplung der Benzotrichloride gemäß der allgemeinen Formel (II). Vorzugsweise wird Pyridin als Lösungmittel verwendet.

Bevorzugt werden Benzotrichloride der allgemeinen Formel (I) eingesetzt, bei denen X Fluor, Chlor oder Nitro bedeutet. Besonders bevorzugt sind Benzotrichloride der allgemeinen Formel (I), bei denen X für Fluor oder Chlor steht. Insbesondere werden 4-Fluorbenzotrichlorid, 4-Chlorbenzotrichlorid, 3,4-Dichlorbenzotrichlorid, 3-Trifluormethyl-4-chlorbenzotrichlorid eingesetzt.

Das Kupfer kann in Form von Pulver oder Spänen eingesetzt werden. Hierbei wird ein molares Verhältnis von Kupfer zu Benzotrichlorid der allgemeinen Formel (II) von (0,4 bis 5): 1, vorzugsweise (0,4 bis 1): 1 und besonders bevorzugt 0,5 : 1 eingesetzt. Die Reaktionstemperatur liegt hierbei üblicherweise im Bereich von 0 bis 115°C. Vorzugsweise arbeitet man im Bereich von 40 bis 80°C.

Bei dieser Kreuzkupplung kommt es ausgehend von den Benzotrichloriden der allgemeinen Formel (II) zur Bildung eines Reaktionsgemisches, welches 1,2-Di(4-halophenyl)tetrachlorethane der allgemeinen Formel (III) 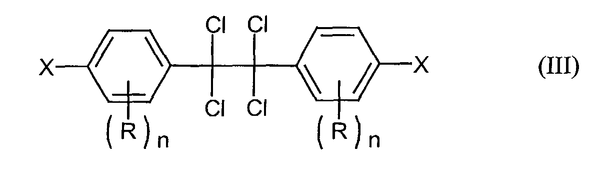 sowie gegebenenfalls 1,2-Di(4-halophenyl)dichlorethylene der allgemeinen Formel (IV) 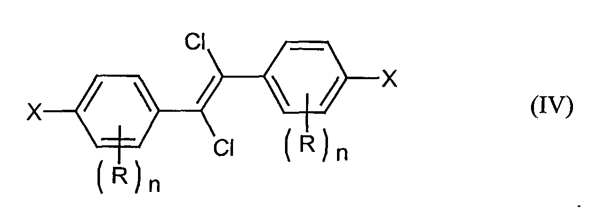 enthält.

Die Aufarbeitung dieses Gemisches kann beispielsweise so erfolgen, dass man es auf Eiswasser austrägt, abfiltriert, den Niederschlag mit Wasser aufschlämmt, frei von Base wäscht (z.B. mit wässriger Chlorwasserstofflösung), abfiltriert und trocknet. Gegebenenfalls kann das so erhaltene Produktgemisch durch Waschen mit einem inerten Lösungsmittel (z.B. Cyclohexan oder Methanol) oder durch Umkristallisieren gereinigt werden. Es könnten jedoch auch andere bekannte Verfahren zur Aufarbeitung genutzt werden.

Gegebenenfalls schließt sich an die Kreuzkupplung der ersten Stufe des erfindungsgemäßen Verfahrens eine Chlorierung des entstandenen Produktgemisches an. Durch diese Chlorierung können die eventuell im Reaktionsgemisch vorliegenden Anteile der 1,2-Di(4-halophenyl)dichlorethylene der allgemeinen Formel (IV) zu den gewünschten 1,2-Di(4-halophenyl)tetrachlorethanen der Formel (III) umgesetzt werden.

Hierzu kann das rohe Reaktionsgemisch der Kreuzkupplung, der eingeengte Rückstand nach dem Waschen mit einem inerten Lösungsmittel oder die eingeengte Mutterlauge nach Kristallisation chloriert werden, um die Verbindungen der Formel (III) in höherer Ausbeute und Reinheit zu erhalten. Die Chlorierung des Gemisches in einem geeigneten Lösungsmittel, vorzugsweise Chloroform, Chlorbenzol oder Chlorbenzotrichlorid, erfolgt gemäß dem Stand der Technik. Das so erhaltene Produkt kann beispielsweise durch Umkristallisieren oder Rühren in einem geeigneten Lösungsmittel, Abfiltrieren und Trocknen gereinigt werden.

Gegenstand der Erfindung sind ferner die Verbindungen der allgemeinen Formel (IV) 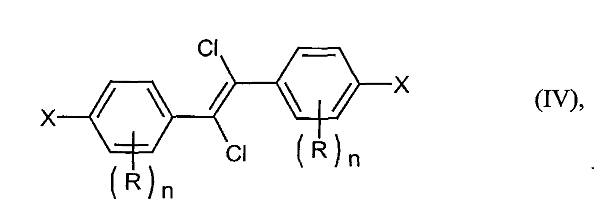 wobei
- X: für Fluor steht und
- R und n: die für die allgemeine Formel (I) genannten Bedeutungen haben.

Die Verbindungen der allgemeinen Formel (IV) können aus dem Produktgemisch von (III) und (IV) beispielsweise durch Destillation isoliert werden.

In einer bevorzugten Ausführungsform erfolgt die Herstellung der Verbindungen der allgemeinen Formel (I) durch die folgende Verfahrenssequenz, indem man
1) ein Benzotrichlorid der allgemeinen Formel (II) in Gegenwart von Kupfer sowie in einem tertiären Amin als Lösungsmittel umsetzt
2) anschließend eine Umsetzung mit wasserfreier Fluorwasserstoffsäure durchführt,
3) danach eine Veretherung mit einem oder mehreren Alkoholen der Formel R³OH und/oder R⁴OH durchführt, und
4) abschließend eine Etherspaltung zu den Verbindungen der allgemeinen Formel (I) vornimmt.

Dieses Verfahren zur Herstellung der Verbindungen der allgemeinen Formel (I) zeichnet sich durch hohe Ausbeuten, leicht zugängliche Ausgangsstoffe und ein geringes Maß an Abfallprodukten aus.

Gegenstand der Erfindung ist ferner die Verwendung der Verbindungen der allgemeinen Formel (I) als Monomere zur Herstellung von Polymeren, bevorzugt Polyestern, Polyethern oder Polycarbonaten, und als Ausgangsstoffe zur Herstellung von Flüssigkristallen oder Flammschutzmitteln.

### Beispiele

### Beispiel 1 (Umsetzung mit 4-Fluorbenzotrichlorid)

3700 g 4-Fluorbenzotrichlorid werden in 14 l Pyridin vorgelegt und bei 65°C portionsweise über 6,5 Stunden mit 565 g Kupfer-Pulver versetzt. Die Mischung wird 16 Stunden bei 65-70°C gerührt. 100 g Kupfer-Pulver werden über 1 Stunde nachdosiert, und die Mischung wird weitere 4 Stunden bei 65-70°C gerührt. Anschließend wird das abgekühlte Reaktionsgemisch auf 60 l Eiswasser gegeben, ausgerührt und abgesaugt. Der Niederschlag wird mit Wasser aufgeschlämmt, mit 1N Chlorwasserstofflösung sauer gestellt und abgesaugt, mit Wasser gewaschen und bei 60°C im Trockenschrank getrocknet.

Erhalten werden 2660 g 1,2-Di(4-fluorphenyl)tetrachlorethan in 84 %iger Reinheit (GC-Flächen %), entsprechend einer Ausbeute von 72 % der Theorie.

Zur weiteren Reinigung wird der Feststoff zweimal mit 2 l Cyclohexan gewaschen und erneut abgesaugt. Erhalten werden 1928 g 1,2-Di(4-fluorphenyl)tetrachlorethan als weißer Feststoff mit einem Schmelzpunkt von 128-130°C. Dies entspricht einer Ausbeute von 62 % der Theorie.

### Beispiel 2 (Umsetzung mit 4-Chlorbenzotrichlorid)

920 g 4-Chlorbenzotrichlorid werden in 3200 ml Pyridin vorgelegt und bei 65-70°C portionsweise mit 128 g Kupfer-Pulver versetzt. Die Mischung wird 19 Stunden bei 70°C gerührt. Anschließend wird das abgekühlte Reaktionsgemisch auf 15 l Eiswasser gegeben, ausgerührt und abgesaugt. Der Niederschlag wird mit Wasser aufgeschlämmt, mit 1N Chlorwasserstofflösung sauer gestellt und abgesaugt, mit Wasser gewaschen und bei 60°C im Trockenschrank getrocknet.

Erhalten werden 587 g 1,2-Di(4-chlorphenyl)tetrachlorethan als weißer Feststoff mit einem Schmelzpunkt von 180-187°C. Dies entspricht einer Ausbeute von 75 % der Theorie.

### Beispiel 3 (Umsetzung mit 3,4-Dichlorbenzotrichlorid)

794 g 3,4-Dichlorbenzotrichlorid werden in 2400 ml Pyridin vorgelegt und bei Raumtemperatur mit 96 g Kupfer-Pulver versetzt. Die Mischung wird 17 Stunden bei 65-70°C gerührt. Anschließend wird das abgekühlte Reaktionsgemisch auf 10 l Eiswasser gegeben, ausgerührt und abgesaugt. Der Niederschlag wird mit Wasser aufgeschlämmt, mit 1N Chlorwasserstofflösung sauer gestellt und abgesaugt, mit Wasser gewaschen und bei 60°C im Trockenschrank getrocknet. Der so entstandene Feststoff wird aus Toluol umkristallisiert.

Erhalten werden 305 g 1,2-Di(3,4-dichlorphenyl)tetrachlorethan als weißer Feststoff mit einem Schmelzpunkt von 197-198°C erhalten. Dies entspricht einer Ausbeute von 44 % der Theorie.

### Beispiel 4 (Umsetzung mit 3-Trifluormethyl-4-chlorbenzotrichlorid)

Analog zu Beispiel 3 wird aus 3-Trifluormethyl-4-chlorbenzotrichlorid das 1,2-Di(3-trifluormethyl-4-chlorphenyl)-tetrachlorethan mit einer Ausbeute von 63 % erhalten.

### Beispiel 5 (Nachchlorierung)

4187 g eines Gemischs aus 1,2-Di(4-fluorphenyl)dichlorethylen (65 %) und 1,2-Di(4-fluorphenyl)tetrachlorethan (31 %) werden in 10,5 l Chloroform vorgelegt. Chlorgas wird durch die Lösung geleitet; gleichzeitig wird das Reaktionsgemisch bei einer Wellenlänge von 254 nm bestrahlt und die Temperatur auf 60°C erhöht. Unter diesen Bedingungen wird sechsmal je 7-8 Stunden photochloriert. Danach wird Stickstoff durch das abgekühlte Reaktionsgemisch geleitet, anschließend wird eingeengt.

Erhalten werden 4580 g 1,2-Di(4-fluorphenyl)tetrachlorethan in einer Reinheit von 87 % (GC-Flächen %), entsprechend einer Ausbeute von 87 % der Theorie.

Zur weiteren Reinigung wird der Feststoff zweimal mit Cyclohexan gewaschen und erneut abgesaugt.

Erhalten werden 3280 g 1,2-Di(4-fluorphenyl)tetrachlorethan in einer Reinheit von 98,6 % (GC-Flächen%) als weißer Feststoff. Dies entspricht einer Ausbeute von 71 % der Theorie.

### Beispiel 6 (Fluorierung mit wasserfreier Flusssäure)

In einem Edelstahlautoklaven werden 600 ml wasserfreie Flusssäure bei Raumtemperatur vorgelegt. Anschließend werden 571 g 1,2-Di(4-fluorphenyl)tetrachlorethan zugegeben. Die Temperatur wird in Stufen auf 146°C erhöht und der entstehende Chlorwasserstoff über einen Rückflusskühler (-15°C) bei 10 bis 35 bar entspannt. Nach Ende der HCl-Gas-Entwicklung wird auf Normaldruck entspannt und die überschüssige Flusssäure abdestilliert. Der Rückstand wurde in Dichlormethan gelöst und mit Aktivkohle versetzt, dann filtriert. Das Filtrat wird eingeengt, dann bei 16 mbar destilliert (Siedepunkt bei 16 mbar: 125-126°C).

Erhalten werden 370 g 1,2-Di(4-fluorphenyl)tetrafluorethan als weißer Feststoff mit einem Schmelzpunkt von 96-97°C. Dies entspricht einer Ausbeute von 80 % der Theorie.

### Beispiel 7 (Fluorierung mit wasserfreier Flusssäure)

In einem Edelstahlautoklaven werden 1000 ml wasserfreie Flusssäure und 25 ml Antimon(V)chlorid bei Raumtemperatur vorgelegt. Anschließend werden 1157 g 1,2-Di(4-chlorphenyl)tetrachlorethan zugegeben. Die Temperatur wirde analog zu Beispiel 5 in Stufen auf 140°C erhöht. Nach Ende der HCl-Gas-Entwicklung wird entspannt und die überschüssige Flusssäure abdestilliert. Der Rückstand wird Petrolether umkristallisiert.

Erhalten werden 467 g 1,2-Di(4-chlorphenyl)tetrafluorethan als weißer Feststoff mit einem Schmelzpunkt von 88-90°C erhalten. Dies entspricht einer Ausbeute von 49 % der Theorie.

### Beispiel 8 (Fluorierung mit wasserfreier Flusssäure)

In einem Edelstahlautoklaven werden 500 ml wasserfreie Flusssäure und 5 ml Antimon(V)chlorid bei Raumtemperatur vorgelegt. Anschließend werden 229 g 1,2-Di(3,4-dichlorphenyl)tetrachlorethan zugegeben. Die Temperatur wird in Stufen auf 146°C erhöht. Nach Ende der HCl-Gas-Entwicklung wird entspannt und die überschüssige Flusssäure abdestilliert. Der Rückstand wird in Toluol aufgenommen und mit Aktivkohle und Natriumfluorid versetzt, dann filtriert. Das so erhaltene Filtrat wird eingeengt und dann in Methanol umkristallisiert.

Erhalten werden 467 g 1,2-Di(3,4-dichlorphenyl)tetrafluorethan als weißer Feststoff mit einem Schmelzpunkt von 105-107°C. Dies entspricht einer Ausbeute von 49 % der Theorie.

¹H-NMR (d₆-DMSO, 400 MHz): [δ in ppm] 7.67 (2 H, d, *J* 8.4 Hz), 7.65 (2 H, d, *J* 2.2 Hz), 7.40 (2 H, dd, *J* 8.4 und 2.2 Hz); ¹⁹F-NMR (d₆-DMSO, 376 MHz): [δ in ppm] -111 (4 F, s); MS (EI) 392 (10 %) [M⁺], 195 (100) [Cl₂C₆H₃-CF₂⁺].

### Beispiel 9 (Fluorierung mit wasserfreier Flusssäure)

In einem Edelstahlautoklaven werden 1000 ml wasserfreie Flusssäure und 30 ml Antimon(V)chlorid bei Raumtemperatur vorgelegt. Anschließend werden 350 g 1,2-Di(3-trifluormethyl-4-chlorphenyl)tetrachlorethan zugegeben. Stickstoff wird aufgedrückt, die Temperatur in Stufen auf 120°C erhöht und der entstehende Chlorwasserstoff über einen Rückflusskühler (-15°C) bei 10 bis 35 bar entspannt. Nach 3 Stunden ist die HCl-Gas-Entwicklung zuende, es wird entspannt und die überschüssige Flusssäure bis 100 mbar abdestilliert. Der Rückstand wird auf Wasser gegeben, dann abgesaugt und im Trockenschrank getrocknet.

Erhalten werden 275 g 1,2-Di(3-trifluormethyl-4-chlorphenyl)tetrafluorethan als weißer Feststoff mit einem Schmelzpunkt von 154-157°C erhalten. Dies entspricht einer Ausbeute von 90 % der Theorie.

MS (EI) 458 (3 %)[M⁺], 229 (100)[Cl-C₆H₃(-CF₃)-CF₂⁺].

### Beispiel 10 (Veretherung mit Benzylalkohol)

216 g Benzylalkohol werden in 1 l N,N-Dimethylacetamid vorgelegt und auf 0°C gekühlt. 336 g Kaliumhydroxid-Pulver wird in einem Zeitraum von 25 Min. bei 0-2°C portionsweise eingetragen. Anschließend wird eine Lösung von 290 g 1,2-Di(4-fluorphenyl)tetrafluorethan in 500 ml N,N-Dimethylacetamid in einem Zeitraum von 45 Min. bei 2-14°C zugetropft. Es wird 30 Minuten bei 14-25°C, dann 16 h bei 84-92°C gerührt. Die resultierende Suspension wird auf 2 l Wasser gegeben, der Feststoff werden abgesaugt, mit Wasser gewaschen und bei 60°C getrocknet.

Erhalten werden 445 g 1,2-Di(4-benzyloxyphenyl)tetrafluorethan in einer Reinheit von 93,4 % (HPLC-Flächen %) als weißer Feststoff mit einem Schmelzpunkt von 206-208°C erhalten. Dies entspricht einer Ausbeute von 95 % der Theorie.

¹H-NMR (d₆-DMSO, 400 MHz): [δ in ppm] 7,62-7,36 (18H, m); 5,33 (4H, s); ¹⁹F-NMR (d₆-DMSO, 376 MHz): [δ in ppm] -109 (4F, s); MS (CI) 489 (100 %) [M+Na⁺], 447 (83) [M⁺-F].

Bei Einsatz von 2 kg 1,2-Di(4-fluorphenyl)tetrafluorethan werden bei entsprechender Vorgehensweise 3,13 kg 1,2-Di(4-benzyloxyphenyl)tetrafluorethan als weißer Feststoff mit einem Schmelzpunkt von 206-208°C erhalten. Dies entspricht einer Ausbeute von 97 % der Theorie.

### Beispiel 11 (Veretherung mit Benzylalkohol)

42,1 g KOH (85-90 %ig, Plätzchen) wird unter einer Stickstoffatmosphäre bei Raumtemperatur in N-Methylpyrrolidon (300 ml) vorgelegt. Die Rührung wird nach Zugabe von 100 ml N-Methylpyrrolidon eingeschaltet. Nach vollständiger Zugabe wird auf 100°C erhitzt. Anschließend werden über 30 Minuten 100 g 1,2-Di(3,4-dichlorphenyl)tetrafluorethan, dann über 10 Minuten 26,4 ml Benzylalkohol zugetropft. Es wird 20 Stunden bei 100°C nachgerührt, dann werden 180 ml Lösungsmittel über eine 10 cm Vigreux/Brücke abdestilliert. Der verbleibende Rückstand wird mit 500 ml Wasser aufgeschlämmt, dann abgesaugt. Der Feststoff wird mit Wasser gewaschen (3 mal mit je 500 ml). Der Rückstand wird im Umlufttrockenschrank bei 70°C getrocknet.

Erhalten werden 113,5 g 1,2-Di(3-chlor-4-benzyloxyphenyl)tetrafluorethan als weißer Feststoff. Dies entspricht einer Ausbeute von 83 % der Theorie.

¹H-NMR (d₆-DMSO, 400 MHz): [δ in ppm] 7,85-7,36 (16H, m) 5,31 (4H,s); ¹⁹F-NMR (d₆-DMSO, 376 MHz): [δ in ppm] -109 (4F,s) ; MS (EI): 534 (2 %) [M⁺], 91(100) [C₆H₅CH₂⁺].

### Beispiel 12 (Veretherung mit Benzylalkohol)

36,0 g KOH (85-90 %ig, Plätzchen) wird unter einer Stickstoffatmosphäre bei Raumtemperatur in N-Methylpyrrolidon (200 ml) vorgelegt. Die Rührung wird nach Zugabe von 100 ml N-Methylpyrrolidon eingeschaltet. Nach vollständiger Zugabe wird auf 100°C erhitzt. Anschließend werden 100 g 1,2-Di(3-trifluormethyl-4-chlorphenyl)tetrafluorethan in N-Methylpyrrolidon (50 ml), dann 22,5 ml Benzylalkohol zugetropft. Es wird 42 h bei 100°C nachgerührt, dann werden 90 ml Lösungsmittel über eine 10 cm Vigreux/Brücke abdestilliert. Der verbleibende Rückstand wird mit Wasser aufgeschlämmt, dann abgesaugt. Der Feststoff wird mit Wasser gewaschen (3 x 200 ml). Der Rückstand wird im Umlufttrockenschrank bei 70°C getrocknet.

Erhalten werden 49 g 1,2-Di(3-trifluormethyl-4-benzyloxyphenyl)tetrafluorethan als weißer Feststoff erhalten. Dies entspricht einer Ausbeute von 53 % der Theorie.

¹H-NMR (d₆-DMSO, 400 MHz): [δ in ppm] 7,83 (2H, d, *J* 9,0 Hz); 7,56-7,36 (16H, m); 5,38 (4H, s); ¹⁹F-NMR (d₆-DMSO, 376 MHz): [δ in ppm] -61 (6F, s), -109 (4F, s); MS (EI) (1 %) [M⁺], 91 (100) [C₆H₅CH₂⁺].

### Beispiel 13 (Etherspaltung durch Hydrierung)

2866 g 1,2-Di(4-benzyloxyphenyl)tetrafluorethan werden in 28 l Ethanol vorgelegt und mit 280 g 5 %igem Palladium auf Aktivkohle versetzt. Anschließend wird bei 25-30°C über einen Zeitraum von 15 Stunden ein Druck von 2-4 bar Wasserstoff aufgedrückt, dann wird entspannt. Das Gemisch wird abgesaugt und das Filtrat eingeengt.

Erhalten werden 1718 g 1,2-Di(4-hydroxyphenyl)tetrafluorethan in einer Reinheit von 98,4 % (HPLC-Flächen %) als weißer Feststoff mit einem Schmelzpunkt von 224-225°C erhalten. Dies entspricht einer Ausbeute von 98 % der Theorie.

¹H-NMR (d₆-DMSO, 400 MHz): [δ in ppm] 7,18 (4H, d, *J* 8,6); 6,85 (4H, d, *J* 8,6); ¹⁹F-NMR (d₆-DMSO, 376 MHz): [δ in ppm] -109 (4F, s); MS (EI) 286 (14 %) [M⁺], 143 (100) [HO-C₆H₄-CF₂⁺].

### Beispiel 14 (Etherspaltung durch Hydrierung)

113 g 1,2-Di(3-chlor-4-benzyloxyphenyl)tetrafluorethan werden in 500 ml Ethanol vorgelegt und mit 1 g 5 %igem Palladium auf Aktivkohle versetzt. Anschließend wird bei 25-30°C über einen Zeitraum von 30 Stunden ein Druck von 2-4 bar Wasserstoff aufgedrückt, dann wird entspannt. Das Gemisch wird heiß filtriert und das Filtrat eingeengt.

Erhalten werden 45 g 1,2-Di(3-chlor-4-hydroxyphenyl)tetrafluorethan als weißer Feststoff. Dies entspricht einer Ausbeute von 60 % der Theorie.

¹⁹F-NMR (d₆-DMSO, 376 MHz): [δ in ppm] -110 (4F,s); MS (CI) 355 (100 %) [M+H⁺].

### Beispiel 15 (Etherspaltung durch Hydrierung)

100 g 1,2-Di(3-trifluormethyl-4-benzyloxyphenyl)tetrafluorethan werden in 500 ml Ethanol vorgelegt und mit 1 g 5 %igem Palladium auf Aktivkohle versetzt. Anschließend wird bei 25-30°C über einen Zeitraum von 10 Stunden ein Druck von 2-4 bar Wasserstoff aufgedrückt, dann wird entspannt. Das Gemisch wird filtriert und das Filtrat eingeengt.

Erhalten werden 29 g 1,2-Di(3-trifluormethyl-4-hydroxyphenyl)tetrafluorethan als weißer Feststoff in einer Reinheit von 98,9 % (GC-Flächen %) erhalten. Dies entspricht einer Ausbeute von 85 % der Theorie.

¹H-NMR (d₆-DMSO, 400 MHz): [δ in ppm] 11,45 (2H, s); 7,58 (2H, d, *J* 8,6 Hz); 7,38 (2H, s); 7,20 (2H, d, *J* 8,6 Hz); ¹⁹F-NMR (d₆-DMSO, 376 MHz): [δ in ppm] -61 (6F, s), -110 (4F, s); MS (EI) 422 (12 %) [M⁺], 211 (100) [HO-C₆H₃(-CF₃)-CF₂⁺].

## Patentansprüche

1. Verbindungen der allgemeinen Formel (I) 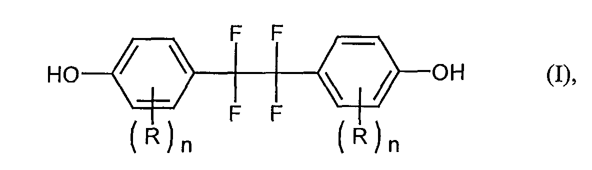 worin
R unabhängig voneinander Wasserstoff, F, Cl, Br, I, CN, COOR², C₁-C₄-Alkyl, C₁-C₄-Alkoxy, C₁-C₄-Alkylthio, C₁-C₄-Perfluoralkyl, C₁-C₄-Perfluoralkoxy, C₁-C₄-Perfluoralkylthio, C₁-C₄-Polyfluoralkyl, C₁-C₄-Polyfluoralkoxy und C₁-C₄-Polyfluoralkylthio bedeuten können,
R² C₁-C₄-Alkyl ist und
n eine ganze Zahl von 0 bis 4 ist.

2. Verbindungen nach Anspruch 1, **dadurch gekennzeichnet, dass**
R unabhängig voneinander Wasserstoff, F, Cl, Br, Methyl, Methoxy, Trifluormethyl oder Trifluormethoxy bedeuten können und
n 0 oder 1 ist.

3. 1,2-Di(4-hydroxyphenyl)tetrafluorethan, 1,2-Di(3-chlor-4-hydroxyphenyl)-tetrafluorethan, 1,2-Di(3-fluor-4-hydroxyphenyl)tetrafluorethan, 1,2-Di(3-brom-4-hydroxyphenyl)tetra-fluorethan und 1,2-Di(3-methyl-4-hydroxy-phenyl)tetrafluorethan.

4. Verfahren zur Herstellung der Verbindungen der allgemeinen Formel (I) gemäß Anspruch 1, **dadurch gekennzeichnet ist, dass** Verbindungen der allgemeinen Formel (VI) 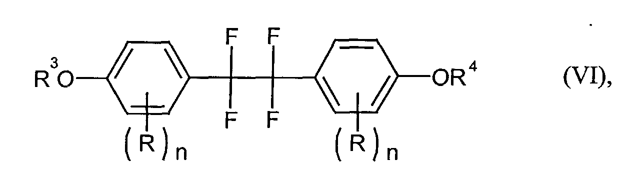 worin
R³ und R⁴ gleich oder verschieden sind und Benzyl, substituiertes Benzyl, bevorzugt 1-(C₁-C₄-Alkyl)benzyl, Benzhydryl, substituiertes Benzhydryl, iso-Propyl, tert-Butyl oder Cyclohexyl darstellen und
R und n die gleiche Bedeutung besitzen wie in der allgemeinen Formel (I),
einer Etherspaltung unterworfen werden.

5. Verfahren nach Anspruch 4, **dadurch gekennzeichnet, dass** die Etherspaltung der Verbindungen der allgemeinen Formel (VI) über eine Hydrierung oder eine Spaltung im sauren Milieu erfolgt.

6. Verfahren nach Anspruch 5, **dadurch gekennzeichnet, dass** eine Hydrierung erfolgt, wenn R³ und/oder R⁴ einen Benzylrest oder substituierten Benzylrest darstellen, und eine Spaltung im sauren Milieu erfolgt, wenn R³ und/oder R⁴ iso-Propyl, tert-Butyl oder Cyclohexyl darstellen.

7. Verbindungen der allgemeinen Formel (VI) 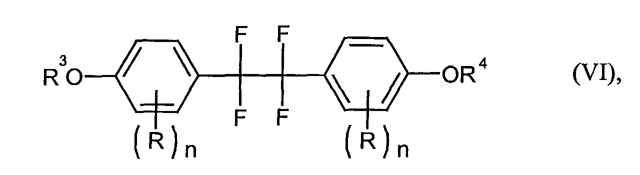 worin
R³ und R⁴ gleich oder verschieden sind und Benzyl, substituiertes Benzyl, bevorzugt 1-(C₁-C₄-Alkyl)benzyl, Benzhydryl, substituiertes Benzhydryl, iso-Propyl, tert-Butyl oder Cyclohexyl darstellen und
R und n die gleiche Bedeutung besitzen wie in der allgemeinen Formel (I).

8. Verfahren zur Herstellung von Verbindungen der allgemeinen Formel (VI), **dadurch gekennzeichnet, dass** Verbindungen der allgemeinen Formel (V) 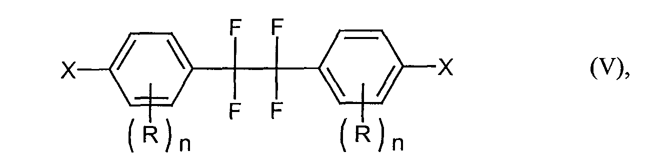 worin
X für ein Halogen oder Pseudohalogen und
R und n die für die allgemeine Formel (I) genannten Bedeutungen besitzen,
mit einem Alkohol der Formel R³OH und/oder einem Alkohol der Formel R⁴OH umgesetzt werden, wobei R³ und R⁴ die für die allgemeine Formel (VI) in Anspruch 7 genannten Bedeutungen besitzen.

9. Verfahren zur Herstellung von Verbindungen der allgemeinen Formel (V), **dadurch gekennzeichnet, dass** Verbindungen der allgemeinen Formel (III) 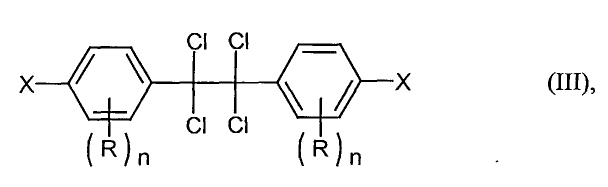 worin
X die für die allgemeine Formel (V) in Anspruch 8 genannten Bedeutungen besitzt und
R und n die für die allgemeine Formel (I) genannten Bedeutungen besitzen,
mit wasserfreier Fluorwasserstoffsäure umgesetzt werden.

10. Verbindungen der allgemeinen Formel (IV) 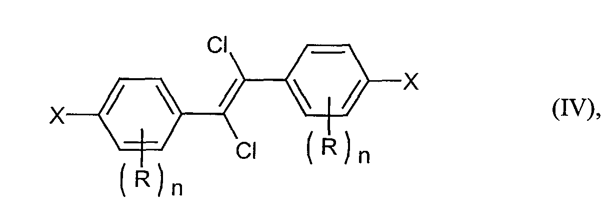 wobei
X für Fluor steht und
R und n die für die allgemeine Formel (I) genannten Bedeutungen besitzen.

11. Verfahren zur Herstellung von Verbindungen der allgemeinen Formel (I) 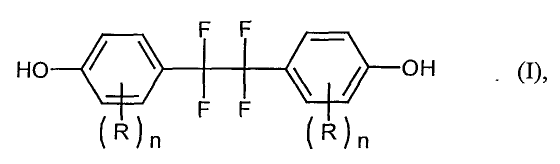 worin
R unabhängig voneinander Wasserstoff, F, Cl, Br, I, CN, COOR², C₁-C₄-Alkyl, C₁-C₄-Alkoxy, C₁-C₄-Alkylthio, C₁-C₄-Perfluoralkyl, C₁-C₄-Perfluoralkoxy, C₁-C₄-Perfluoralkylthio, C₁-C₄-Polyfluoralkyl, C₁-C₄-Polyfluoralkoxy und C₁-C₄-Polyfluoralkylthio bedeuten können,
R² C₁-C₄-Alkyl ist und
n eine ganze Zahl von 0 bis 4 ist,
wobei
1) ein Benzotrichlorid der allgemeinen Formel (II) 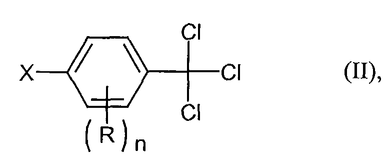 worin
R und n die für die allgemeine Formel (I) angegebenen Bedeutungen besitzen und
X ein Halogen oder Pseudohalogen ist, in Gegenwart von Kupfer sowie in einem tertiären Amin als Lösungsmittel umgesetzt wird,
2) eine Umsetzung mit wasserfreier Fluorwasserstoffsäure durchgeführt wird,
3) danach eine Veretherung mit einem Alkohol der Formel R³OH und/oder einem Alkohol der Formel R⁴OH durchgeführt wird,
wobei
R³ und R⁴ gleich oder verschieden sind und Benzyl, substituiertes Benzyl, bevorzugt 1-(C₁-C₄-Alkyl)benzyl, Benzhydryl, substituiertes Benzhydryl, iso-Propyl, tert-Butyl oder Cyclohexyl darstellen und
4) abschließend eine Etherspaltung zu den Verbindungen der allgemeinen Formel (I) vorgenommen wird.

12. Verwendung der Verbindungen der allgemeinen Formel (I) als Monomere zur Herstellung von Polymeren, bevorzugt Polyestern, Polyethern oder Polycarbonaten, und als Ausgangsstoffe zur Herstellung von Flüssigkristallen oder Flammschutzmitteln.
